# EUROPEAN PATENT APPLICATION

(11) **EP 2 574 330 A1**
(43) Date of publication of application: **03.04.2013**
(21) Application number: 11183548.4
(22) Date of filing: 30.09.2011
(51) Int. Cl.: A61K 8/64, A61Q 19/00, A61Q 19/08, A61K 38/01

(54) **Composition for topical application comprising a protein hydrolysate, applicator and use**

(71) Applicant: Vita Laser B.V., 2451 GG Leimuiden (NL)
(72) Inventor: Farid, Kazem, 2451 GG Leimuiden (NL)
(74) Representative: Jansen, Bart Antonius Johannes

(57) **Abstract**

The invention relates to a composition for topical application, and an applicator and use thereof. The composition for topical application comprises a protein hydrolysate, and optionally other active ingredients and excipients. Test persons treated with protein hydrolysates, applied topically in a cream or lotion, indicated a rejuvenating effect on the skin. The rejuvenating effect is believed to stem from a stimulation of cell growth, in particular the stimulation of fibroblasts.

## Description

### FIELD OF THE INVENTION

The invention relates to a composition for topical application, and an applicator and use thereof.

### BACKGROUND OF THE INVENTION

The cosmetics industry offers many creams and other compositions for treating the skin, such as creams and lotions. The efficacy of known products may vary, and may also depend on the skin of the particular person to be treated.

Placental extracts derived from animal placenta, typically derived from domestic animals such as cow, have been used in several cosmetic products. A desirable effect provided by placenta extracts are the revitalisation and rejuvenation of the skin. Unfortunately, placenta derived from animals may be contaminated and are associated with health risks when used on humans. Also, some persons have shown allergic reactions when treated with placental extracts. Hence, there is a demand for alternative products that provide the benefits of placental extracts but with lower health risks involved.

### OBJECT AND SUMMARY OF THE INVENTION

It is an object of the invention to provide alternative products for skin treatment. It is another object of the invention to provide a skin product to stimulate the skin's natural process of cellular rejuvenation.

The invention provides a composition for topical application comprising a protein hydrolysate, and optionally other active ingredients and excipients. Test persons treated with protein hydrolysates, applied topically in a cream or lotion, indicated a rejuvenating effect on the skin. The rejuvenating effect is believed to stem from a stimulation of cell growth, in particular the stimulation of fibroblasts. The formulations as described herein appear to aid in wound healing. Further, little or none irritating effects on the skin were observed, an indication of having a relatively low allergic potential. It is also believed the formulations as described herein improve the defences of the skin, possible through an increase in lymphocyte multiplication. The composition for topical application is preferably a liquid emulsion or solution, for instance a cream, serum, lotion or gel. Protein hydrolysates may be derived from various protein sources, for instance animal or plant proteins. Hydrolysates are hydrolyzed proteins, broken down into peptides of a relatively small size by enzymatic or chemical methods known in the art. Typically, the size of the peptides in a protein hydrolysate have an average size below 20 kDa.

The formulation may also comprise optional excipients, such as mineral and organic fillers. Examples of suitable additional fillers that may be used in the composition according to the present disclosure include zinc oxide, talc, mica, titanium dioxide, kaolin, bismuth oxychloride, starch, calcium carbonate, magnesium carbonate, barium sulphate, and metal salts derived from organic carboxylic acids comprising from 6 to 22 carbon atoms, for instance zinc stearate, magnesium stearate, zinc myristate, and magnesium myristate.

Optionally, the composition according to the present disclosure may also comprise one or more functional compound such as moisturizers, preservatives, dyes, pigments, fragrances, physical and chemical sun blocking compounds, moisturizers and pH regulators such as acids and bases, preferably forming a buffered system.

It is preferred if the protein hydrolysate is of marine origin, preferably marine animal origin, such as fish, crustaceans and shellfish. Protein hydrolysates of marine origin were anticipated to be less prone to give allergic reactions on the human skin when applied topically and appear to contain a relatively low amount of potentially allergenic components. Compared to protein products, in particular placental extracts, derived from domestic animals such as cattle or pigs, protein hydrolysates of marine origin also are believed to impose a lower health risk, for instance due to contamination by certain microorganisms, priones and/or viruses that are potentially harmful to humans.

It is advantageous if the protein hydrolysate is derived from fish protein. Fish protein has a good availability and was found to be relatively easy to process and hydrolyse. Preferably, a purified protein hydrolysate is used, wherein other substances such as fats and oils are removed. This allows for more reliable and repeatable processing of the proteins. Fish protein hydrolysates may be derived from various types of fish. Fish protein hydrolysates are believed to have a particularly good stimulating effect on fibroblast production, often even better than placental extracts. An additional advantage found for fish protein hydrolysate is that it was relatively easy to obtain an essentially white or colourless formulation. Hydrolysates from other sources where found to be more prone to undesirable coloration.

In a preferred embodiment, the protein hydrolysate is derived from black pollack (Pollachius virens). Black pollack has a good availability and processability, and yielded excellent results when used in cosmetic products. An additional advantage for protein hydrolysates derived from black pollack was that a matting effect on many common skin types. This allows a user to obtain a desired matte visual appearance of the skin with only one skin product, without the need for additional creams or other products for the desired matting effect.

In a preferred embodiment, the protein hydrolysate comprises low molecular weight polypeptides with an average weight below 10.000 Daltons, preferably in the range of 10.000-1000 D, most preferably in the range of 2200-2500 Daltons. Such polypeptides were found to be relatively easy to process for cosmetics, and showed satisfactory cosmetic results in topical application.

It is advantageous if the composition comprises at least 0.1% w/w protein hydrolysate, preferably at least 3% w/w, most preferably from 3-5% w/w. At such concentrations, the cosmetic effect was found to be particularly notable. Above concentrations of 5%, the further increase in effect was found to be limited; therefore the range between 3-5% w/w is believed to give an optimal use of the protein hydrolysate.

In a preferred embodiment, the composition also comprises a dermal delivery system, preferably a liposomal dermal delivery system. Dermal delivery systems compatible with protein hydrolysate give a synergistic effect and improves the cosmetic effect. It is postulated this effect is achieved by improving penetration into the skin by the peptides in the protein hydrolysate. A liposomal dermal delivery system may comprise phospholipids and other lipid-like components.

Examples of delivery systems include liposomes, mixed liposomes, oleosomes, microparticles, nanoparticles, cyclodextrins, vesicles, micelles, mixed micelles, microspheres, nanospheres, lipospheres, microcapsules and nanocapsules, as well as microemulsions and nanoemulsions. Particularly suitable dermal delivery systems include commercially available nanotechnology systems based on liposomes or liposome-like systems, including products marketed as cerasomes and/or rovisomes.

Preferably, the dermal delivery system for protein hydrolysate comprises glycolipids, preferably glycosphingolipids. Glycolipids and in particular glycosphingolipids showed a good compatibility and improved delivery of protein hydrolysate components, resulting in an improved cosmetic effect. They also improve dispersion of protein hydrolysate in emulsion formulations.

The composition may also comprise at least one water soluble polysaccharide or polysaccharide derivative, or a mixture thereof. Polysaccharides improve the moisturizing effect of the composition on the skin. Polysaccharides may also contribute to obtaining a suitable texture for the composition. Suitable polysaccharides include starches, vegetable gums, and pectin. Starches include cornstarch, katakuri starch, potato starch, sago, tapioca and chemically modified derivatives. Vegetable gums comprise alginin, guar gum, locust bean gum, and xanthan gum.

It is particularly advantageous if the composition comprises glycosaminoglycans. Glycosaminoglycans are unbranched polysaccharides and derivatives thereof, comprising repeating disaccharide units, and have shown to stabilize protein hydrolysate in compositions according to the disclosure. Glycosaminoglycans include chondroitin sulfate, dermatan sulfate, keratan sulfate, heparin, heparan sulfate and hyaluronic acid, and their derivatives.

It is also advantageous if the composition comprises hyaluronic acid or derivatives thereof. Hyaluronic acid (also known as hyaluronan or hyaluronate) was shown to have synergetic moisturizing effect in compositions based on protein hydrolysate.

Preferably, the composition as described herein is formulated as a cream, serum, lotion or gel. Such a composition can be prepared from the appropriate ingredients.

The invention also provides to a topical applicator comprising the composition as described herein. A topical applicator makes it easy to apply the composition to the skin in a controlled fashion. Suitable topical applicators include pens, tubes, plunger, sponge and roller applicators.

The invention provides the use of a protein hydrolysate, in particular a fish protein hydrolysate, in the preparation of a topical cosmetic composition as described herein.

The invention also provides the use of a protein hydrolysate, in particular a fish protein hydrolysate, in a topical cosmetic treatment of the human skin. This may for instance be in beauty therapy or in the treatment of the skin after surgery, in particular plastic surgery and the treatment of scar tissue.

### DESCRIPTION OF PREFERRED EMBODIMENTS

The invention will now be further elucidated by the following non-limiting examples.

For the formulations shown in the examples, a commercially available fish protein hydrolysate derived from black pollack was used. This purified protein hydrolysate has an average molecular weight of about 2300 kD. Unless stated otherwise, the compositions were prepared using known mixing techniques.

### Example 1: Day cream

This is a cream for use on the skin, in particular for daily use. The cream is prepared by regular techniques for mixing the active ingredients with a commercially available basic cream.

| **Composition** | **% w/w** |
|---|---|
| Fish protein hydrolysate | 5 |
| Polyethoxylated alcohol (wax) | 5 |
| Sodium lauryl sulphate (surfactant) | 2 |
| Jojoba oil | 1 |
| 2,4-dihydroxybenzophenone | 5 |
| Cyclopentasiloxane/cyclohexasiloxane | 13 |
| Octyl stearate | 9 |
| glycerine | 3 |
| Lecithin (phospholipids) | 3 |
| Hyaluronic acid | 0.5 |
| glycosphingolipids | 3 |
| perfume | 0.3 |
| Benzoic acid | 0.1 |
| water | Up to 100% |

The cream is used by applying and massaging the cream onto the skin. The cream provides a calming, moisturizing and protecting effect to the skin. Users reported a more youthful, radiant and healthy skin after prolonged use. 2,4-dihydroxybenzophenone was added to the day cream as a sunscreen to protect the active ingredients as well as the treated skin from degradation by UV radiation present in sun light.

### Example 2: Night cream

| **Composition** | **% w/w** |
|---|---|
| Fish protein hydrolysate | 5 |
| Polyethoxylated alcohol (wax) | 5 |
| Polysorbate 60 (emulgator) | 3 |
| avocado oil | 3 |
| Vitamin E acetate | 0.3 |
| Octyl stearate | 9 |
| glycerine | 3 |
| Lecithin (phospholipids) | 3 |
| Hyaluronic acid | 0.2 |
| perfume | 0.3 |
| D-panthenol | 0.1 |
| water | Up to 100% |

The cream is used by applying and massaging the cream onto the skin. The skin receives a healing, recharging and restoring effect. Users reported a well balanced, soothed and youthful skin after prolonged use during several weeks.

### Example 3: Serum

| **Composition** | **% w/w** |
|---|---|
| Fish protein hydrolysate | 5 |
| Polyethoxylated alcohol (wax) | 2 |
| polyacrylamide emulsifier | 2 |
| Octyl stearate | 15 |
| glycerine | 2 |
| Lecithin (phospholipids) | 3 |
| Hyaluronic acid | 5 |
| Sirtuin proteins | 3 |
| perfume | 0.3 |
| Benzoic acid | 0.1 |
| water | Up to 100% |

The serum has a lower viscosity and provides a more liquid composition than the creams, that can be applied as drops that can be spread over the skin surface by sweeping and massaging. The serum provides a filling and skin-tightening effect. Users reported a firmer, more vital and younger skin appearance.

The sirtuin proteins or sir2 proteins appeared to have a reviving and activating influence on the skin in a synergetic combination with the fish protein hydrolysate.

### Example 4: Healing Salve

| **Composition** | **% w/w** |
|---|---|
| Fish protein hydrolysate | 5 |
| Polyethoxylated alcohol (wax) | 5 |
| Sodium lauryl sulphate (surfactant) | 2 |
| Jojoba oil | 1 |
| Shea butter | 1 |
| 2,4-dihydroxybenzophenone | 4 |
| Cyclopentasiloxane/cyclohexasiloxane | 11 |
| Octyl stearate | 10 |
| glycerine | 3 |
| Lecithin (phospholipids) | 3 |
| Hyaluronic acid | 1 |
| glycosphingolipids | 3 |
| perfume | 0.3 |
| Benzoic acid | 0.1 |
| water | Up to 100% |

This cream is particularly suitable for the treatment of scar tissue. The composition calms and assists in recovery of damaged skin tissue. After prolonged use, scars appeared to be less visible and the healing process appeared to be faster.

## Claims

1. Composition for topical application comprising a protein hydrolysate, and optionally other active ingredients and excipients.

2. Composition according to claim 1, wherein the protein hydrolysate is of marine origin, preferably marine animal origin.

3. Composition according to claim 2, wherein the protein hydrolysate is derived from fish protein.

4. Composition according to claim 3, wherein the protein hydrolysate is derived from black pollack (*Pollachius virens*).

5. Composition according to any of the preceding claims, wherein the protein hydrolysate comprises low molecular weight polypeptides with an average weight below 10.000 Daltons, preferably in the range of 10.000-1000 D, most preferably in the range of 2200-2500 Daltons.

6. Composition according to any of the preceding claims, wherein the composition comprises at least 0.1 % w/w protein hydrolysate, preferably at least 3% w/w, most preferably from 3-5% w/w.

7. Composition according to any of the proceeding claims, wherein the composition comprises a dermal delivery system, preferably a liposomal dermal delivery system.

8. Composition according to claim 7, wherein the dermal delivery system comprises glycolipids, preferably glycosphingolipids.

9. Composition according to any of the preceding claims, wherein the composition also comprises at least one water soluble polysaccharide or polysaccharide derivative, or a mixture thereof.

10. Composition according to claim 9, wherein the composition comprises glycosaminoglycans.

11. Composition according to claim 9 or 10, wherein the composition comprises hyaluronic acid or derivatives thereof.

12. Composition according to any of the preceding claims, wherein the composition is formulated as a cream, serum, lotion or gel.

13. Topical applicator comprising the composition according to any of the preceding claims.

14. Use of a protein hydrolysate, in particular a fish protein hydrolysate, in the preparation of a topical cosmetic composition.

15. Use of a protein hydrolysate, in particular a fish protein hydrolysate, in a cosmetic treatment of the human skin.
